# EUROPEAN PATENT APPLICATION

(11) **EP 1 734 032 A1**
(43) Date of publication of application: **20.12.2006**
(21) Application number: 05728046.3
(22) Date of filing: 01.04.2005
(51) Int. Cl.: C07C 67/31, C07C 69/712, G03F 7/039, H01L 21/027

(54) **CALIXRESORCINARENE COMPOUNDS, PHOTORESIST BASE MATERIALS, AND COMPOSITIONS THEREOF**

(30) Priority: 05.04.2004 JP 2004111459; 05.04.2004 JP 2004111460
(71) Applicant: IDEMITSU KOSAN CO., LTD., Tokyo 100-8321 (JP)
(72) Inventor: ISHII, Hirotoshi, 2990293 (JP); OWADA, Takanori, 2990293 (JP); SHIBASAKI, Yuzi; c/o Tokyo Institute of Technology, 1528550 (JP); UEDA, Mitsuru; c/o Tokyo Institute of Technology,, 1528550 (JP)
(74) Representative: Gille Hrabal Struck Neidlein Prop Roos
(86) International application number: PCT/JP2005/006512
(87) International publication number: WO 2005/097725

(57) **Abstract**

A calixresorcinarene compound shown by the following formula (1), 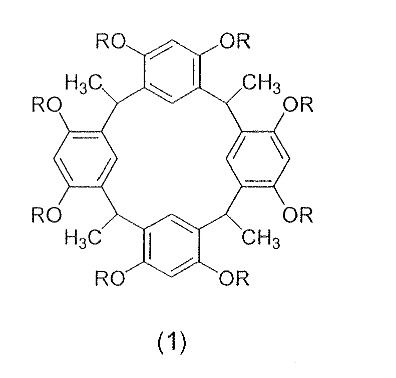 wherein R individually represents a hydrogen atom, a 1-tetrahydropyranyl group, a 1-tetrahydrofuranyl group, or one or more organic groups selected from the group consisting of the organic groups shown by the following formulas, 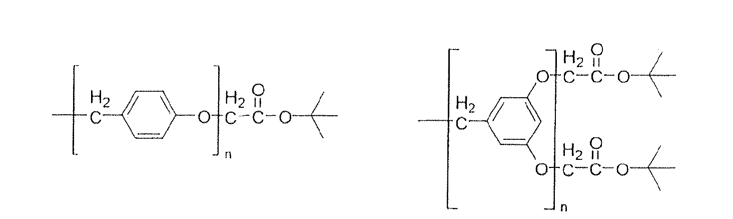 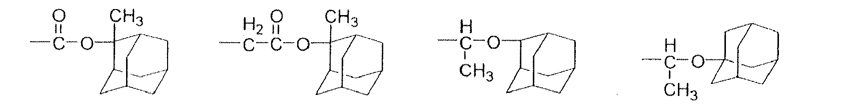 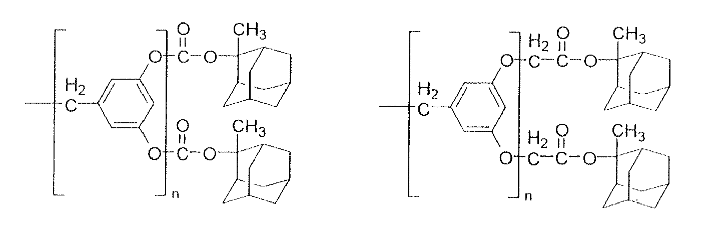 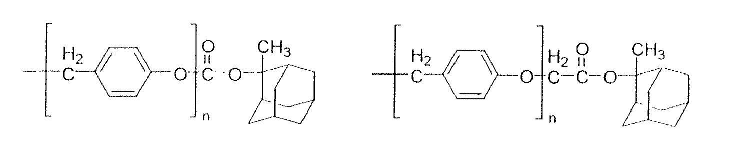 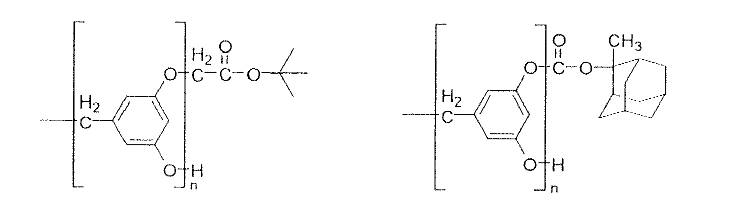 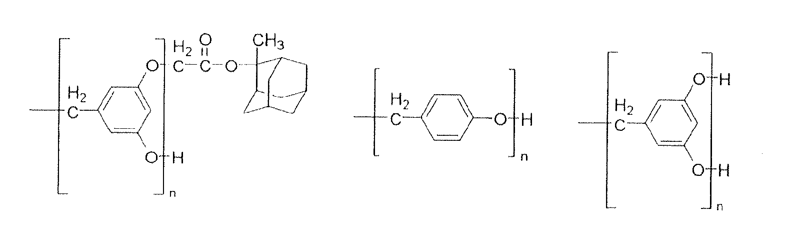 wherein n individually represents an integer of 1 to 50, provided that a compound in which R is selected only from a hydrogen atom, a 1-tetrahydropyranyl group, and a 1-tetrahydrofiiranyl group is excluded.

## Description

### TECHNICAL FIELD

The invention relates to a novel calixresorcinarene compound, a photoresist substrate used in the electricity and the electronic field such as a semiconductor, the optical field, and the like, and a composition of the same.

### BACKGROUND ART

Lithography using extreme ultraviolet radiation (EUV) or an electron beam is useful as a high productivity and high resolution microfabrication method in the manufacture of semiconductors and the like. Development of a high sensitivity and high resolution photoresist used in lithography is desired. Improvement of sensitivity is essential for a photoresist used in the lithographic technique from the viewpoint of productivity of desired detailed patterns, high resolution, and the like.

As a photoresist used for microfabrication using extreme ultraviolet radiation, a chemically-amplified polyhydroxystyrene-based photoresist used for microfabrication using a known KrF laser, for example, can be given. This resist is known to be usable for microfabrication to a degree of about 50 nm. However, if patterns more detail than 50 nm, which is the greatest merit of the microfabrication using extreme ultraviolet radiation, are produced using this resist, problems such as low sensitivity, large line edge roughness, and a large amount of resist out-gas occur. The resist thus cannot necessarily sufficiently derive excellent performance inherent to extreme ultraviolet radiation. Therefore, development of a photoresist exhibiting higher performance has been demanded.

In order to respond to such a demand, a method of using a chemically amplified positive-tone photoresist with a higher photoacid generator concentration as compared with other resist compounds has been proposed (e.g. , refer to Patent Document 1). This Patent Document discloses a photoresist comprising a hydroxystyrene/styrene/t-butyl acrylate terpolymer base material, a photoacid generator of which at least about 5 wt% of the total solid content is di(t-butylphenyl)iodonium ortho-trifluoromethylsulfonate, lactate of tetrabutylammonium hydroxide, and ethyl lactate in examples. However, no specific results such as a line width obtained by using extreme ultraviolet radiation are described. Therefore, the results attainable by using this composition have been thought that microfabrication to the extent of 100 nm, shown in an example in which electron beams were used, is a limit in terms of line edge roughness. An overreaction of the basic material due to addition of an excessive amount of photoacid generator, specifically, over-diffusion of acids to non-exposed areas may be a cause of this limitation.
[Patent Document 1] JP-A-2002-055457

The invention has been achieved in view of this situation and has an object of providing a novel calixresorcinarene compound, a photoresist base material enabling ultra microfabrication using extreme ultraviolet radiation and/or an electron beam, while exhibiting high sensitivity, high resolution, and low line edge roughness, and a composition of the same.

The inventors have conducted extensive studies in order to achieve the above object and found that a problem that occurs in microfabrication using a conventional photoresist is a decrease in sensitivity of the photoresist, which is caused by a molecular shape of a high molecular compound conventionally used as the photoresist base material, reactivity due to the structure of protective groups in the molecular structure of the photoresist base material, and basic impurities in residues originating from a reaction agent or catalyst used during preparation of the photoresist base material or basic impurities mixed in the photoresist base material from human bodies or environment.
In particular, a photoacid generator is sometimes used at a high concentration in a photoresist when the absorbance of extreme ultraviolet radiation and an electron beam passing through a photoresist layer is high and the strength of a light source is low. If a small amount of a basic impurity mixes in such a photoresist, such an impurity neutralizes protons generated from the acid generator and a desired reaction does not proceed. This problem is particularly remarkable in a calixresorcinarene-based photoresist.
The inventors have found a photoresist base material free from these problems and exhibiting high sensitivity, high resolution, and low line edge roughness, leading to completion of the invention.

### DISCLOSURE OF THE INVENTION

According to the invention, the following calixresorcinarene compounds and the like are provided.
1. A calixresorcinarene compound shown by the following formula (1), 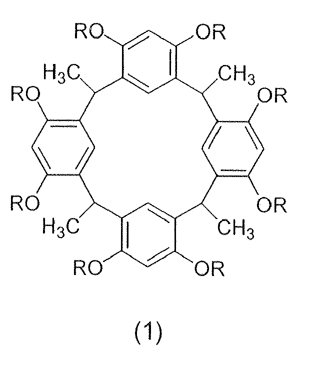 wherein R individually represents a hydrogen atom, a 1-tetrahydropyranyl group, a 1-tetrahydrofuranyl group, or one or more organic groups selected from the group consisting of the organic groups shown by the following formulas, 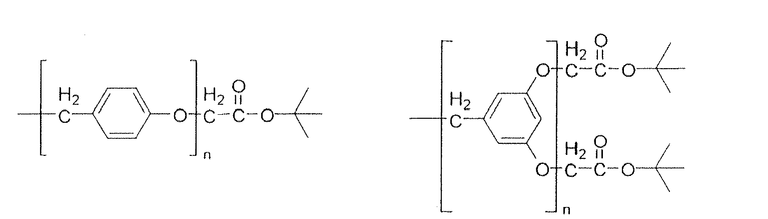 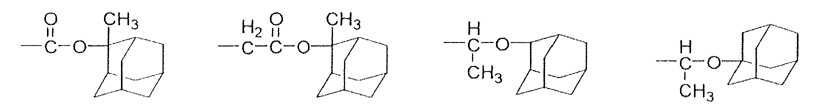 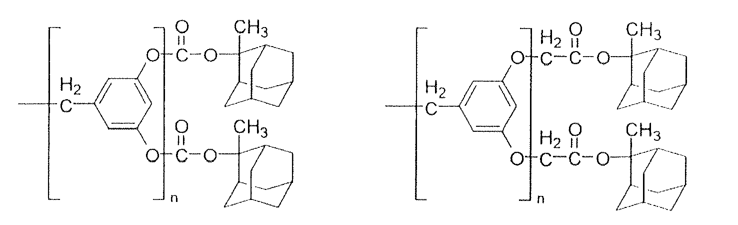 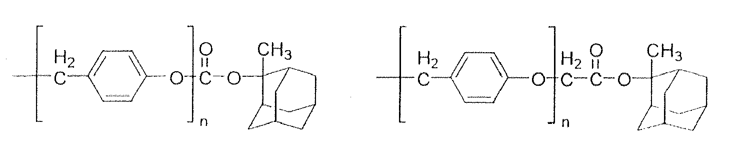 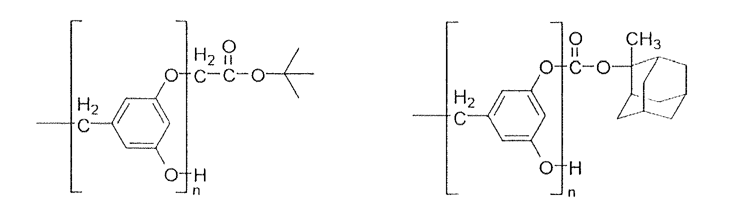 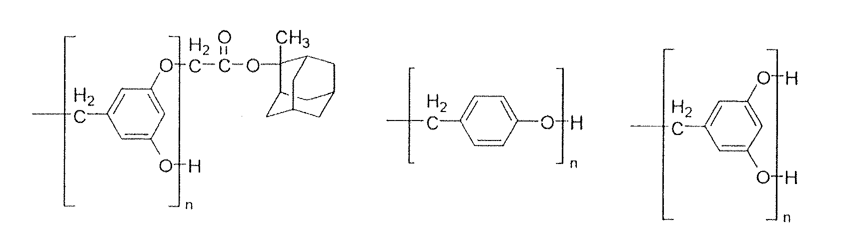 wherein n individually represents an integer of 1 to 50,
   provided that a compound in which R is selected only from a hydrogen atom, a 1-tetrahydropyranyl group, and a 1-tetrahydrofuranyl group is excluded.
2. A purification method of a calixresorcinarene compound comprising washing the compound according to 1 with an acidic aqueous solution and processing the washed compound with an ion-exchange resin.
3. A photoresist base material for extreme ultraviolet radiation and/or an electron beam comprising the calixresorcinarene compound shown by the above formula (1).
4. A photoresist composition for extreme ultraviolet radiation and/or an electron beam comprising the photoresist base material according to 3 and a solvent.
5. The photoresist composition according to 4, further comprising a photoacid generator.
6. The photoresist composition according to 4 or 5, further comprising a basic organic compound as a quenching agent.
7. A photoresist composition comprising a photoresist base material that is an extreme ultraviolet radiation-reactive organic compound shown by the following formula (2), obtained by washing with an acidic aqueous solution and processing with an ion-exchange resin, a photoacid generator or a photobase generator, and a quenching agent, 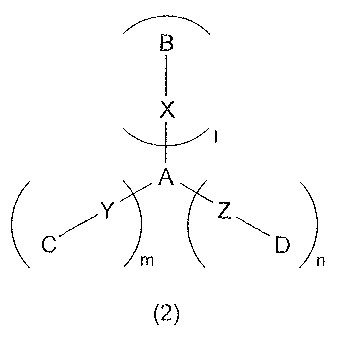 wherein A is an organic group represented by any of the following formulas, 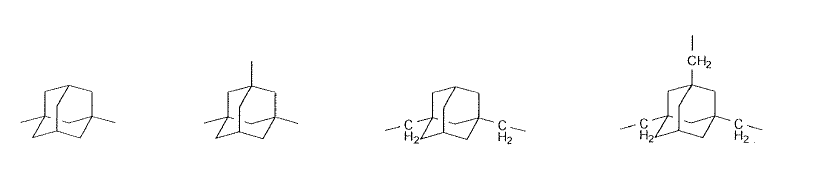 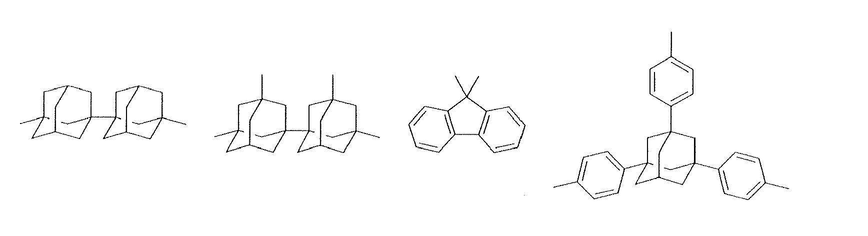 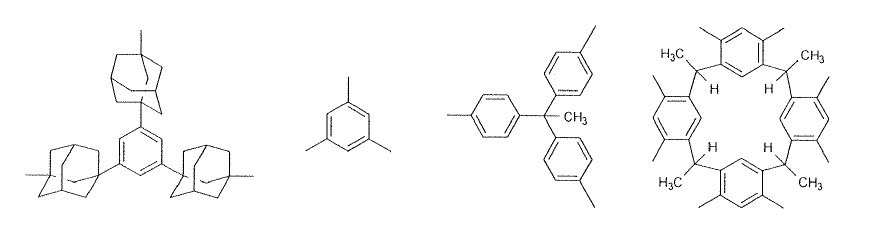 B, C, and D are individually a group reactive with extreme ultraviolet radiation, a group reactive with an effect of a chromophore active to extreme ultraviolet radiation, or an organic group of any of the following formulas, 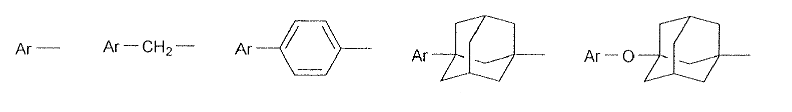 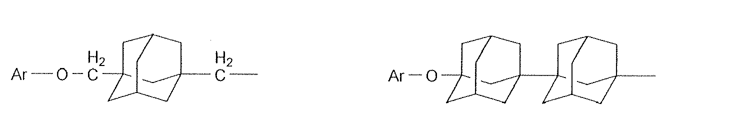 wherein Ar is a phenyl group or a naphthyl group substituted with RO- and/or ROCO-, wherein R, RO-, and ROCO- are groups reactive with extreme ultraviolet radiation or groups reactive with an effect of a chromophore active to extreme ultraviolet radiation,
   X, Y, and Z individually represent a single bond or an ether bond, and 1 + m + n = 2, 3, 4, or 8.
8. The photoresist composition according to 7, wherein the extreme ultraviolet-radiation reactive organic compound is in an amorphous state at room temperature and the average diameter of the molecule is 2 nm or less.
9. The photoresist composition according to 7 or 8, wherein A is an organic group represented by any of the following formulas, 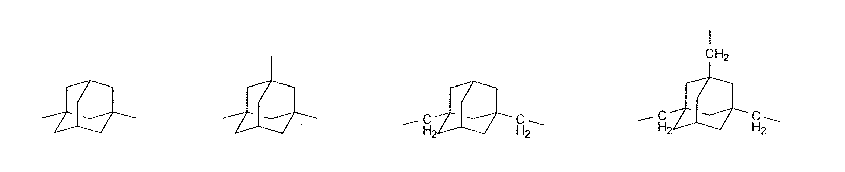 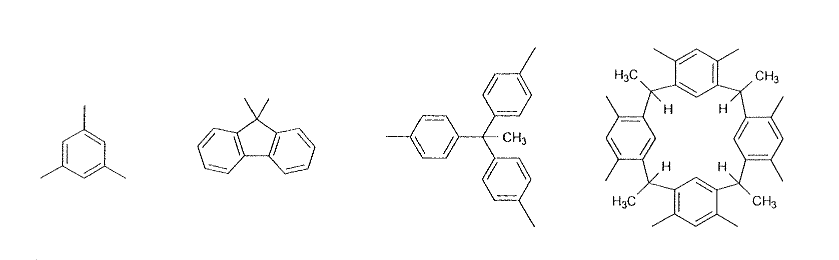 B, C, and D are individually a hydrogen atom, a tert-butyl group, tert-butyloxycarbonylmethyl group, tert-butyloxycarbonyl group, 1-tetrahydropyranyl group, 1-tetrahydrofuranyl group, 1-ethoxyethyl group, 1-phenoxyethyl group, organic group shown by the following formula, 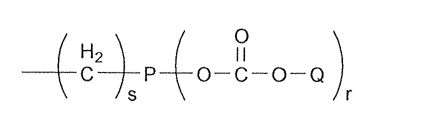 wherein P is an aromatic group having 6 to 20 carbon atoms with a valence of (r + 1), Q represents an organic group having 4 to 30 carbon atoms, r is an integer of 1 to 10, and s is an integer of 0 to 10, or an organic group represented by any of the following formulas, 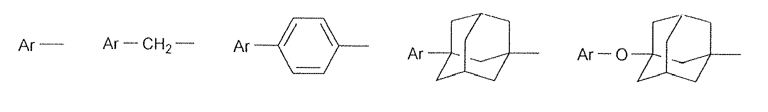 wherein Ar is a phenyl group or a naphthyl group substituted with RO- and/or ROCO-, wherein R is a hydrogen atom, a tert-butyl group, tert-butyloxycarbonylmethyl group, tert-butyloxycarbonyl group, 1-tetrahydropyranyl group, 1-tetrahydrofuranyl group, 1-ethoxyethyl group, 1-phenoxyethyl group, or an organic group shown by the following formula, 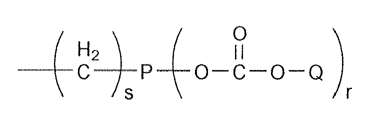 wherein P is an aromatic group having 6 to 20 carbon atoms with a valence of (r + 1), Q represents an organic group having 4 to 30 carbon atoms, r is an integer of 1 to 10, and s is an integer of 0 to 10, and
   X, Y, and Z individually represent a single bond or an ether bond.
10. The photoresist composition according to 7 or 9, wherein A is any one of the organic groups represented by the following formulas, 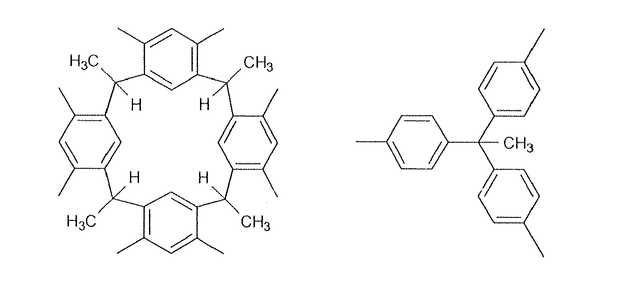 B, C, and D are individually a hydrogen atom, atert-butyl group, tert-butyloxycarbonylmethyl group, tert-butyloxycarbonyl group, 1-tetrahydropyranyl group, 1-tetrahydrofuranyl group, 1-ethoxyethyl group, 1-phenoxyethyl group, or an organic group shown by the following formula, 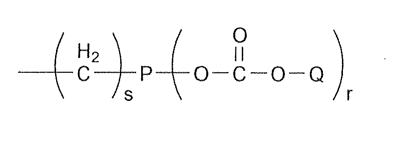 wherein P is an aromatic group having 6 to 20 carbon atoms with a valence of (r + 1), Q represents an organic group having 4 to 30 carbon atoms, r is an integer of 1 to 10, and s is an integer of 0 to 10, and
   X, Y, and Z are ether bonds.
11. A photoresist composition comprising a photoresist base material that is a photosensitive organic compound shown by the following formula (2), obtained by washing with an acidic aqueous solution and processing with an ion-exchange resin, a photoacid generator or a photobase generator, and a quenching agent, 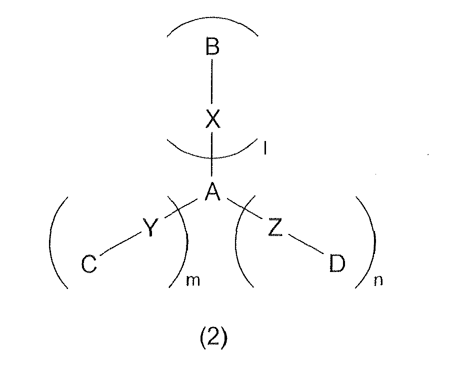 wherein A is an organic group represented by one of the following formulas, 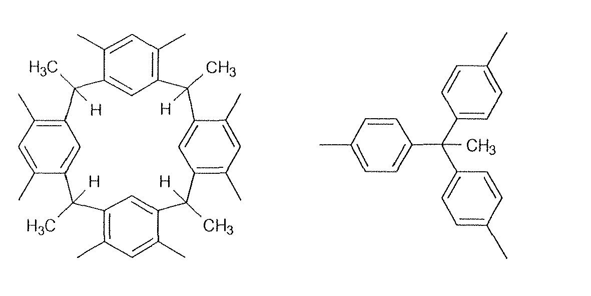 B, C, and D are individually a tert-butyloxycarbonylmethyl group, tert-butyloxycarbonyl group, or an organic group shown by the following formula, 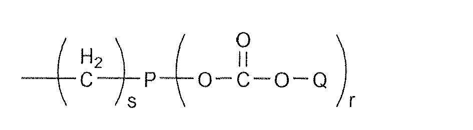 wherein P is an aromatic group having 6 to 20 carbon atoms with a valence of (r + 1), Q represents an organic group having 4 to 30 carbon atoms, r is an integer of 1 to 10, and s is an integer of 0 to 10, and
   X, Y, and Z individually represent a single bond or an ether bond, and 1 + m + n = 3 or 8.
12. The photoresist composition according to 11, wherein the organic group shown by the following formula, 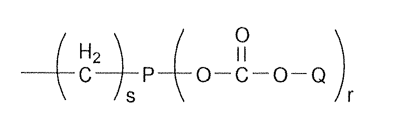 is a 4-(tert-butoxycarbonyloxy)benzyl group or a 3,5-di(tert-butoxycarbonyloxy)benzyl group.
13. The photoresist composition according to 11 or 12, wherein the radiation is extreme ultraviolet radiation or an electron beam.
14. The photoresist composition according to any one of 7 to 13, wherein at least one of B, C, and D is a hydrogen atom and X, Y, and Z are ether bonds.
15. The photoresist composition according to any one of 7 to 14, wherein the basic impurity content of the photoresist base material is not more than 10 ppm.
16. A microfabrication method by lithography using the photoresist composition according to any one of 4 to 6 and 7 to 15.
17. A semiconductor device prepared using the photoresist composition according to any one of 4 to 6 and 7 to 15.

According to the invention, a novel calixresorcinarene compound, a photoresist base material enabling ultra microfabrication using extreme ultraviolet radiation and/or an electron beam, while exhibiting high sensitivity, high resolution, and low line edge roughness, and a composition of the same can be provided.

### BRIEF DESCRIPTION OF THE DRAWINGS

Figure 1 shows a ¹H-NMR spectrum chart of calixresorcinarene compound synthesized in Example 1.
Figure 2 is a line and space pattern of the photoresist composition of Example 2.
Figure 3 is an isolated line pattern of the photoresist composition of Comparative Example 1.
Figure 4 is a line and space pattern of the photoresist composition of Comparative Example 2.

### BEST MODE FOR CARRYING OUT THE INVENTION

The calixresorcinarene compound of the invention is shown by the following formula (1), 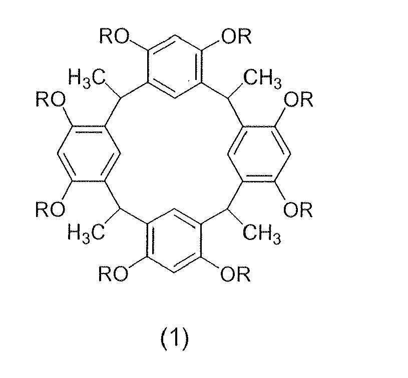 wherein R individually represents a hydrogen atom, a 1-tetrahydropyranyl group, a 1-tetrahydrofuranyl group, or one or more organic groups selected from the group consisting of the organic groups shown by the following formulas, 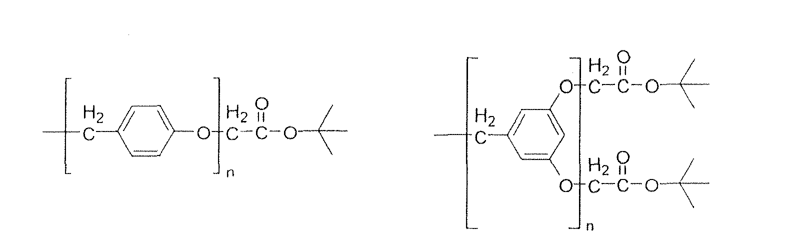 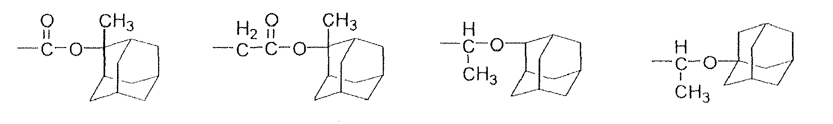 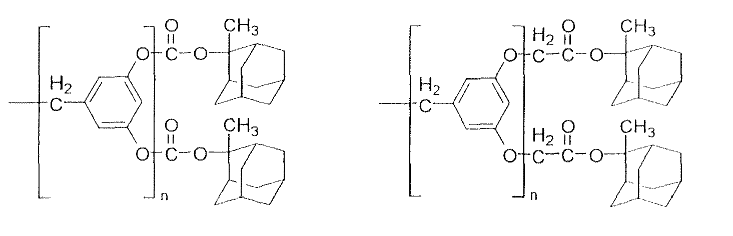 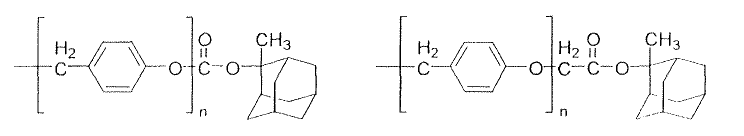 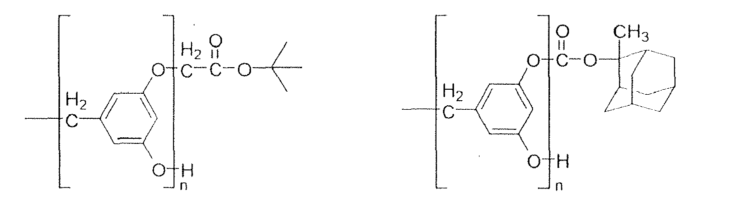 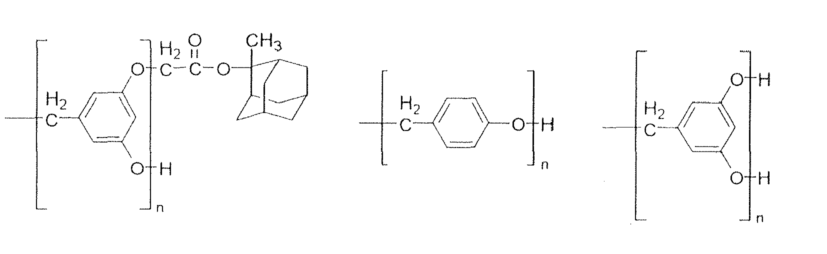 wherein n is individually an integer of 1 to 50, provided that a compound in which R is selected only from a hydrogen atom, a 1-tetrahydropyranyl group, and a 1-tetrahydrofuranyl group is excluded, and provided further that one to seven R groups among eight are preferably hydrogen atoms.

Such a compound is useful as a photoresist base material, particularly, as a photoresist base material used for ultra-microfabrication using extreme ultraviolet radiation or electron beams.

In addition, the organic compound of the following formula (2) can be used as a photoresist base material. These compounds are photosensitive.
Radiation used herein refers to ultraviolet radiation with a wavelength of 10 to 300 nm, particularly, to extreme ultraviolet radiation, vacuum ultraviolet radiation, an electron beam, an ion beam, and the like.

This compound is reactive preferably with extreme ultraviolet radiation and/or an electron beam, and more preferably with an electron beam. In addition, this compound can also react with general radiation other than the above specific types of radiation (for example, infrared radiation, visible rays, ultraviolet radiation (g line, i line, etc.), and X-rays). 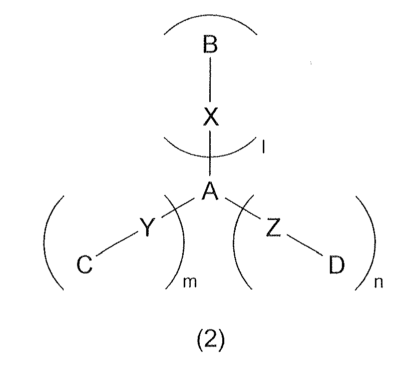 wherein A is an organic group represented by one of the following formulas, 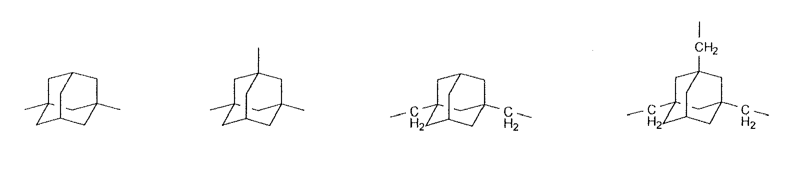 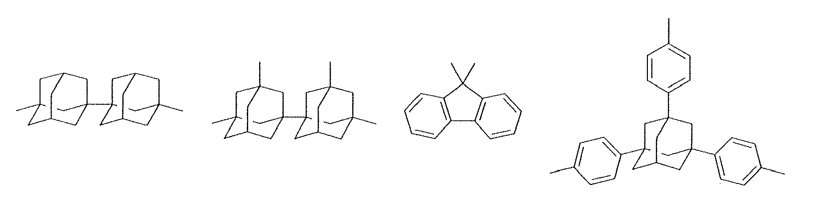 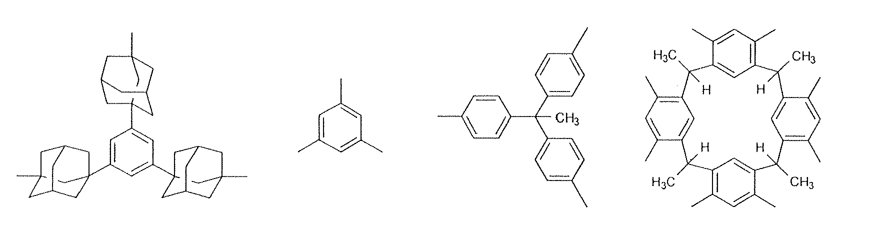 B, C, and D are individually a radiation sensitive group, a group reactive with an effect of a chromophore active to radiation, or an organic group of any of the following formulas, 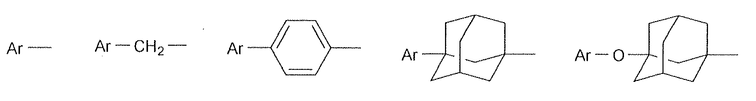 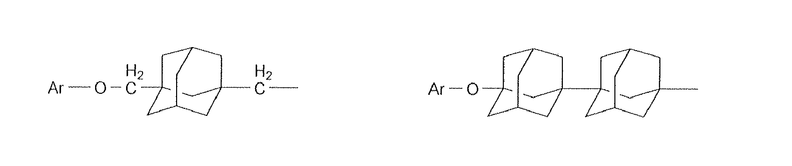 wherein Ar is a phenyl group or a naphthyl group substituted with RO- and/or ROCO-, wherein R, RO-, and ROCO- are groups reactive with a radiation sensitive group or a group reactive with an effect of a chromophore active to radiation,
X, Y, and Z individually represent a single bond or an ether bond, and 1 + m + n = 2, 3, 4, or 8.

B to D are a substituent reactive to irradiation (radiation sensitive group), a group reactive with an effect of a chromophore active to radiation, or a substituent including these groups.
It is preferable that at least one of B, C, and D is a hydrogen atom.

As specific examples of the substituents B to D, organic groups shown below, later-described radiation sensitive groups, and later-described groups reactive with an effect of a chromophore active to radiation (R, RO-, and ROCO-) can be given. 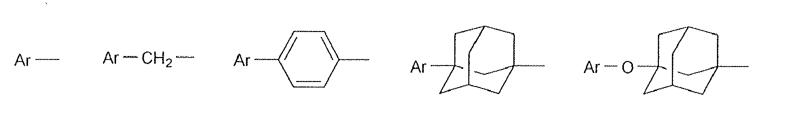 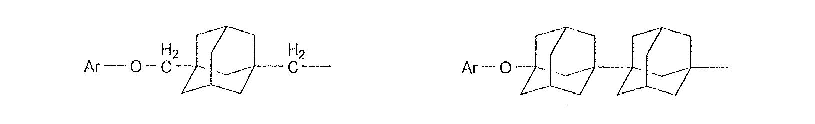 wherein Ar is a phenyl group or a naphthyl group substituted with RO- and/or ROCO-, wherein R, RO-, and ROCO- are groups reactive with a radiation sensitive group or a group reactive with an effect of a chromophore active to radiation.

As specific examples of the compound used in the invention, compounds of the following formulas (11) to (26) and their position isomers can be given.

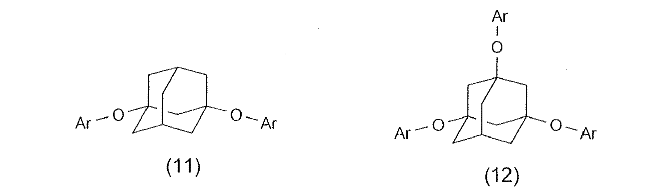

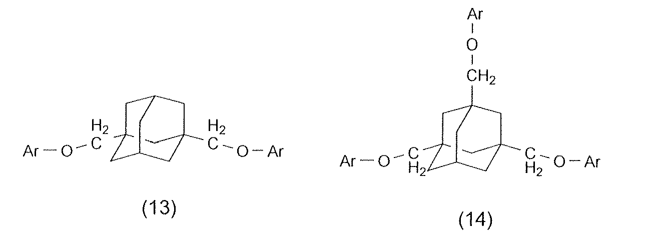

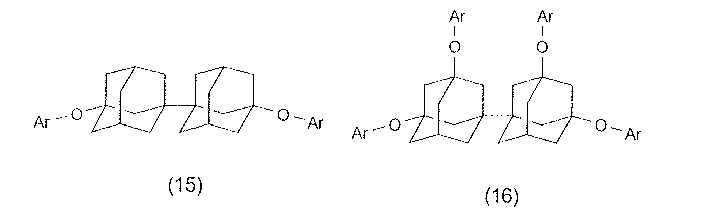

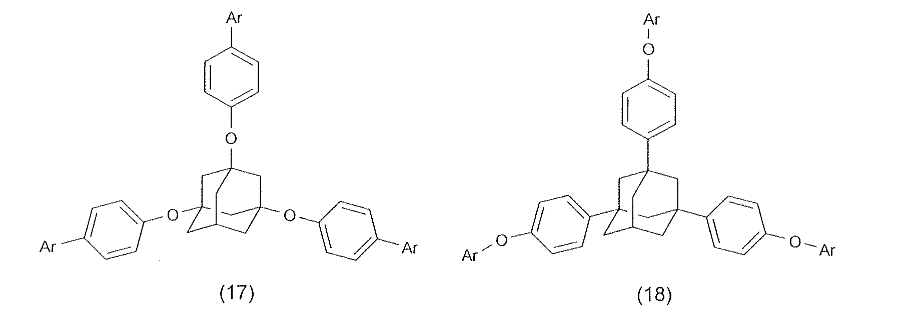

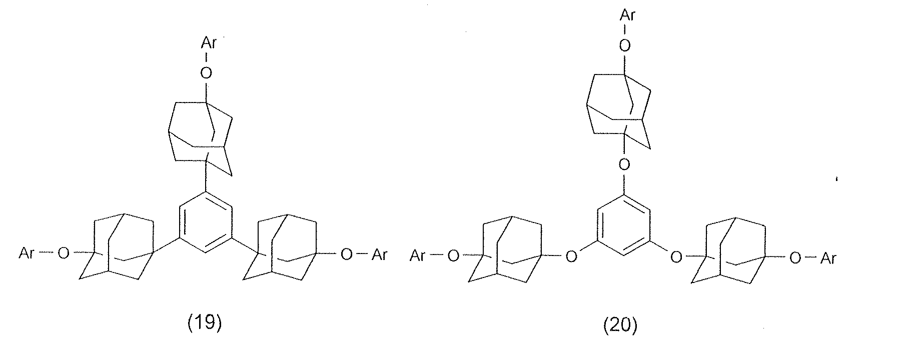

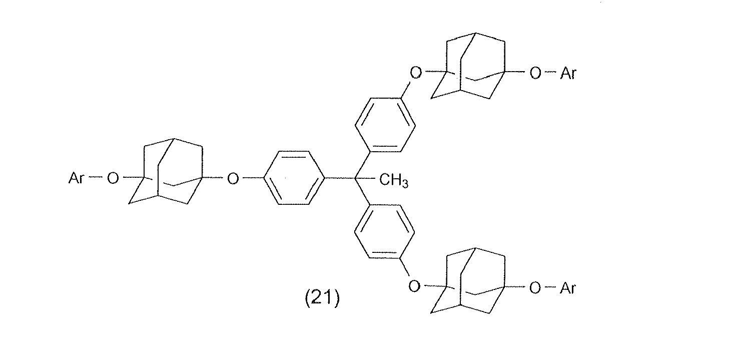

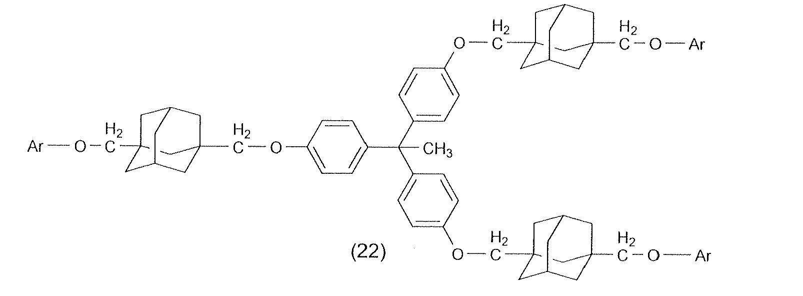

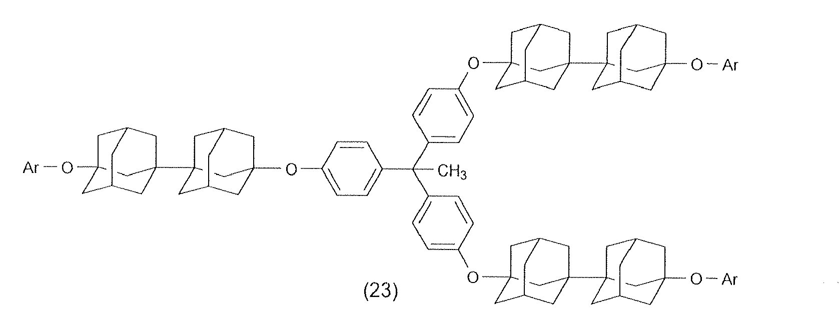

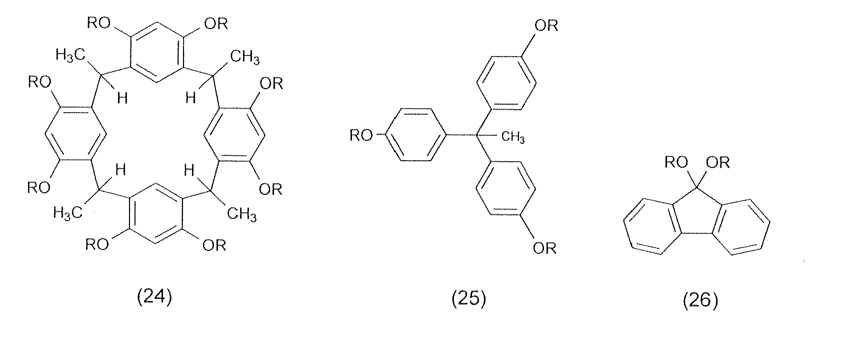

As the substituent Ar in the formulas (11) to (23), any groups containing R, RO-, or ROCO- which is a group containing a radiation sensitive group or a group reactive with an effect of a chromophore active to radiation (described later), can be preferably used. Specifically, substituents shown below, their position isomers, and the like can be given. The groups for the substituent Ar may be used either alone or, to the extent that the effect of the invention is not impaired, two or more groups for the substituent Ar may be used in combination.

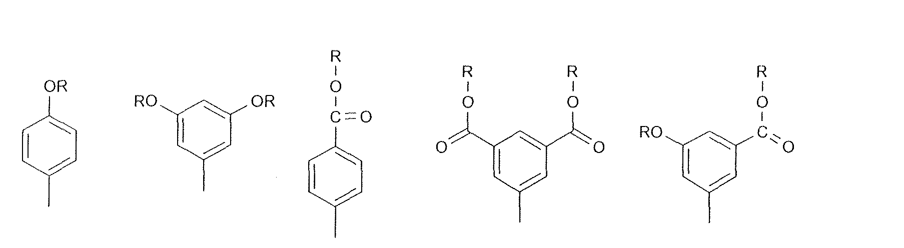

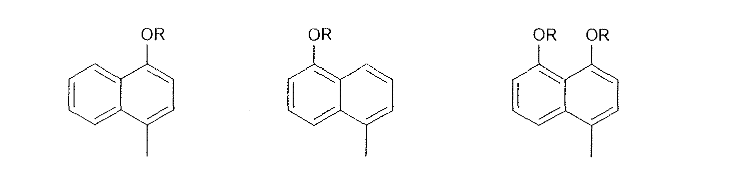

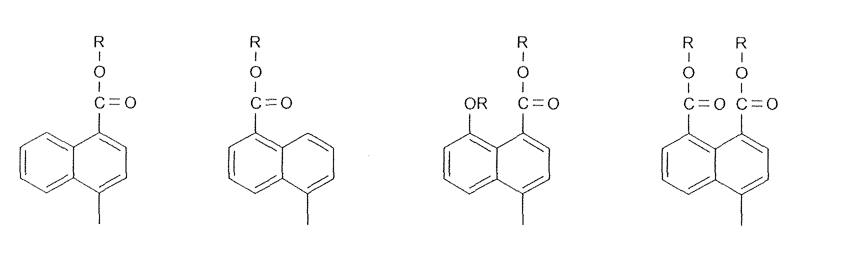

In the formulas (24) to (26), the substituent Ar, and the like, any groups can be preferably used for the substituent R, RO-, and ROCO-, insofar as these groups are radiation sensitive groups or groups reactive with an effect of a chromophore active to radiation. As specific examples of R, a hydrogen atom, tertiary hydrocarbon groups such as a tert-butyl group and an adamantyl group; substituents in which the RO- group forms a carbonic acid ester group such as a tert-butoxycarbonyl group; substituents in which the RO- group forms an acetal group such as a methoxymethyl group, a 1-ethoxyethyl group, and a 1-phenoxyethyl group; and substituents shown below can be given. The substituents R may be used either alone or, to the extent that the effect of the invention is not impaired, two or more groups of R may be used in combination. 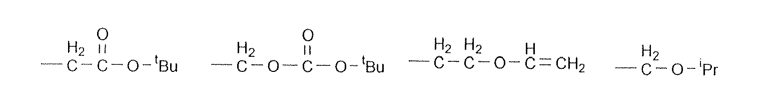 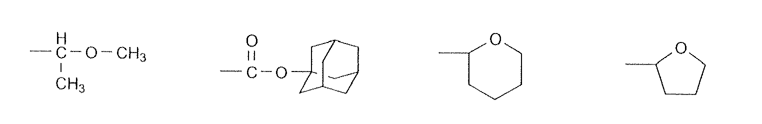 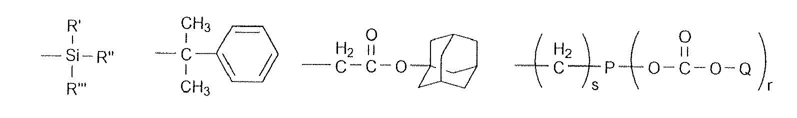 wherein R', R", and R"' individually represent an aliphatic hydrocarbon group having 1 to 10 carbon atoms or an aromatic group, P is an aromatic group having 6 to 20 carbon atoms with a valence of (r + 1), Q represents an organic group having 4 to 30 carbon atoms, r is an integer of 1 to 10, s is an integer of 0 to 10, ^{t}Bu indicates a tert-butyl group, and ⁱPr indicates an isopropyl group.

In the organic group represented by the formula, 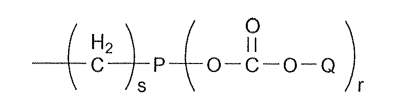 the aromatic group P and the organic group Q preferably have 6 to 10 carbon atoms and 4 to 20 carbon atoms, respectively, and r and s are preferably an integer of 1 to 5 and an integer of 0 to 3, respectively.

Specifically, the following organic groups can be given.

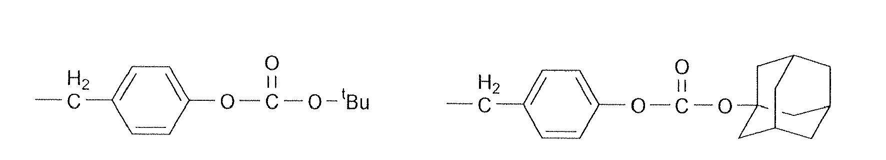

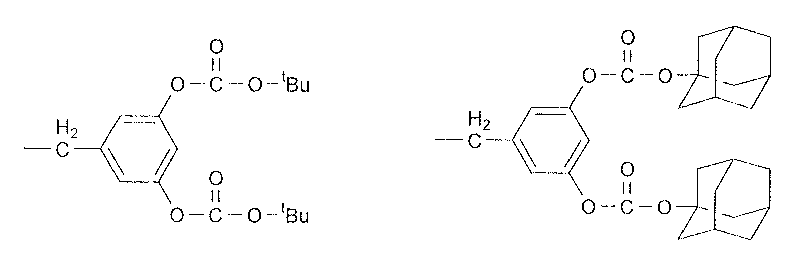

The compound shown by the formula (2) is preferably in an amorphous state at room temperature and the average diameter of the molecule is less than a desired pattern size, preferably 5 nm or less, and more preferably 2 nm or less. The average diameter here is defined as the diameter of a structure obtainable by optimization using the AM1 method of the semi-empirical orbital method program package MOPAC97, assuming that the volume of the space occupied by the structure based on van-der-Waals radius standard is spherical.

The compounds shown by the formulas (1) and (2) used by the invention are synthesized by combining known reactions. In this instance, the compounds obtained by the reaction can be purified by appropriately separating any impurities contained in the compounds using a known method.

Since the compounds used in the invention are in the sate of amorphous under the conditions in which the compounds are used as the photoresist base material, usually at room temperature, the compounds are preferable base materials used in a photoresist composition exhibiting excellent applicability and capability of producing photoresist film with excellent strength.
In addition, the compounds used in the invention usually have an average diameter of the molecule of less than a desired pattern size, specifically, less than the value of line edge roughness required for the size of 100 nm or less, and particularly 50 nm or less. For this reason, if these compounds are used as a base material, the compound can suppress the line edge roughness to 2 nm or less, and preferably 1 nm or less (3σ), when used for processing in a range of 20 to 50 nm, featuring the ultra-microfabrication using extreme ultraviolet radiation or an electron beam.

When the compounds of the invention are used as a base material, the amount of basic impurities, such as ammonia, alkali metal ions (e.g. , Li, Na, K, etc.), alkaline earth metal ions (e.g., Ca, Ba, etc.), and the like, contained in these compounds before purification is preferably reduced to 1/10 or less.
A specific content of basic impurities is preferably 10 ppm or less, and still more preferably 2 ppm or less.
A 10 ppm or less basic impurity content of these compounds can dramatically increase the sensitivity of the photoresist base material to extreme ultraviolet radiation and an electron beam and, as a result, ensures fabrication of minute patterns by lithographic processing of the photoresist composition.

In the invention, the basic impurity content of these compounds can be reduced to 10 ppm or less by purifying these compounds by washing with an acidic aqueous solution, followed by processing with an ion-exchange resin. In this instance, an optimal acidic aqueous solution and ion-exchange resin can be appropriately selected according to the amount and type of the basic impurities to be removed, the type of the compound to processed, and the like. In the invention, an acetic acid aqueous solution with a concentration of 0.01 to 10 mol/liter is preferably used as the acidic aqueous solution and a cation-exchange resin is preferably used as the ion-exchange resin. A particularly preferable method of purification comprises washing with an acetic acid aqueous solution as the acidic aqueous solution, followed by processing with a cation-exchange resin.

These compounds may be used as a photoresist base material either alone or, to the extent that the effect of the invention is not impaired, in combination of two or more. In addition, compounds produced by combining two or more of these compounds by optional substituents may be used either alone or, to the extent that the effect of the invention is not impaired, in combination of two or more.

The photoresist base material of the invention can be used as one of the components of the photoresist composition. The photoresist composition of the invention preferably comprises a solvent in addition to the photoresist base material. The composition of the invention preferably further comprises a photoacid generator or a photobase generator and a quenching agent in addition to the photoresist base material. Preferably, the composition of the invention is a liquid composition comprising these components and a solvent for dissolving these components. It is preferable that the composition is liquid in order to uniformly apply a photoresist to a substrate and the like which are to be processed by ultra-microfabrication.

Since the molecule of the photoresist base material of the invention contains a chromophore active to radiation such as extreme ultraviolet radiation and/or an electron beam, the photoresist base material can exhibit performance as a photoresist by itself. Therefore, it is unnecessary to add an additive. However, if promotion of the performance as a photoresist is desired, a photoacid generator (PAG) or a photobase generator (PBG) can be added as a chromophore, as required.

As the PAG, in addition to known compounds of which the structures are exemplified below, compounds having the same effect can be commonly used. These compounds can be appropriately selected according to the type of substrate, a desired shape, size, and the like of minute patterns. The amount of PAG added is usually in a range of 50 to 0.1 wt% of the total amount of the photoresist base material. 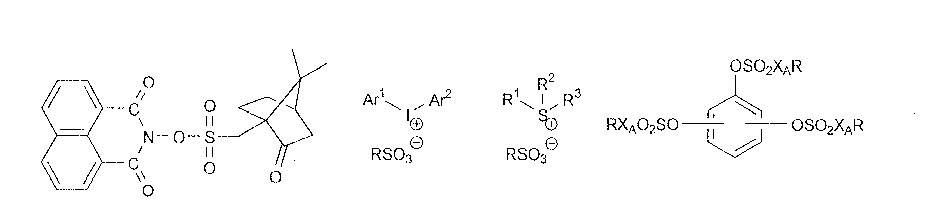 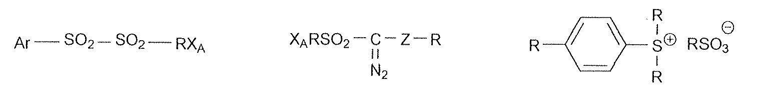 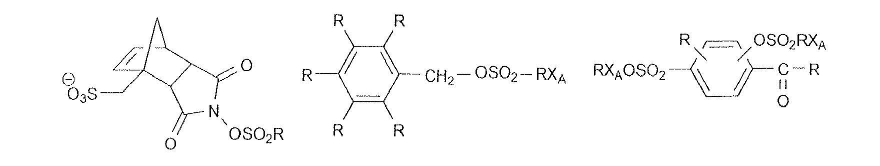 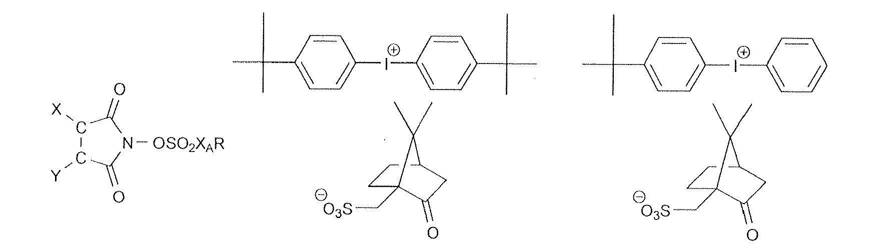 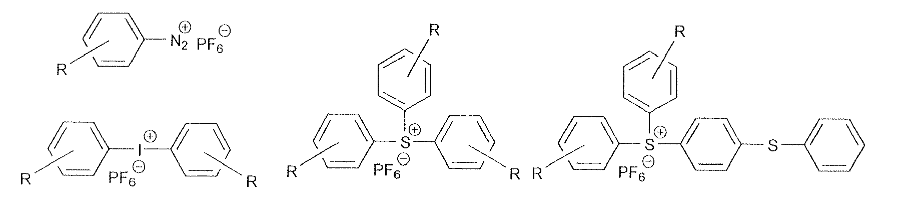 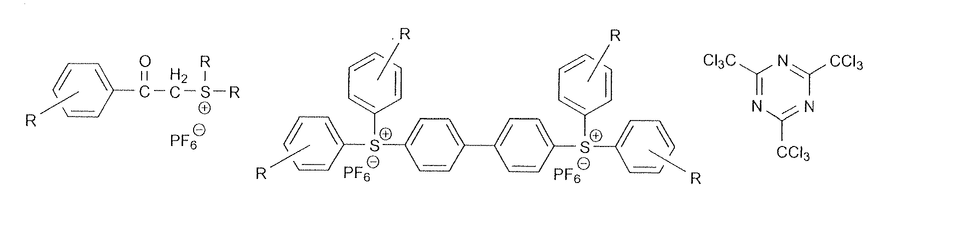 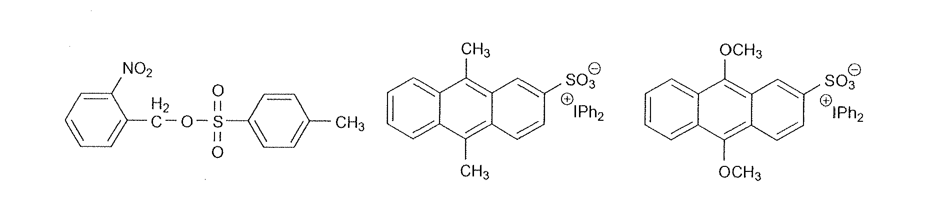 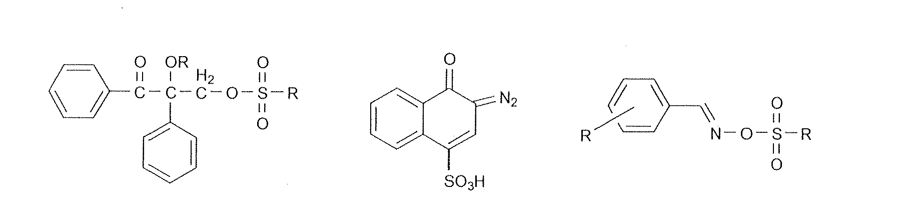 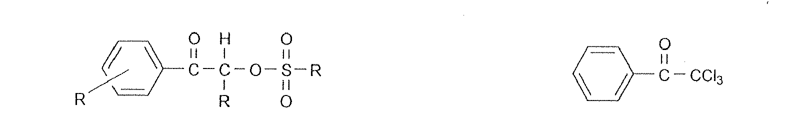 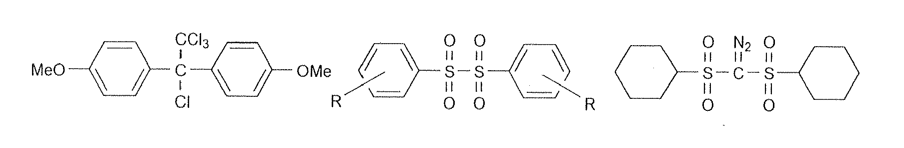 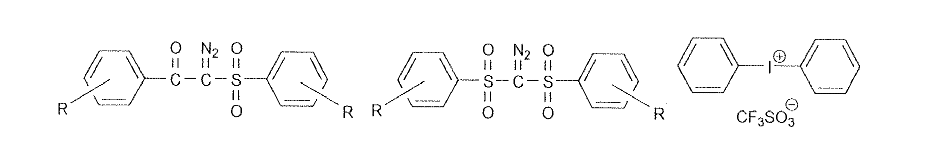 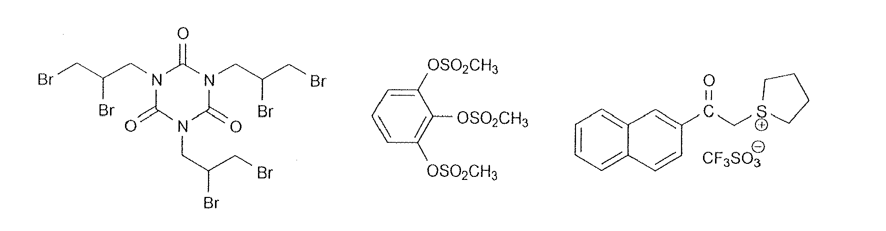 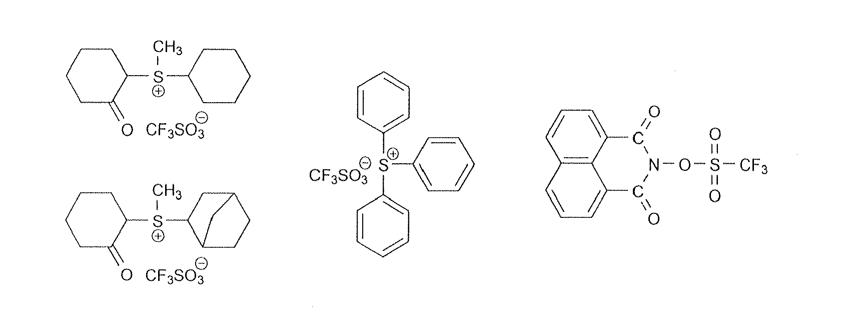 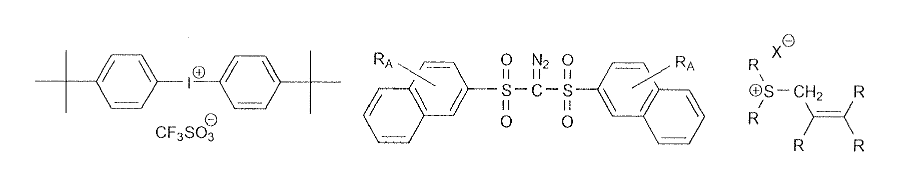 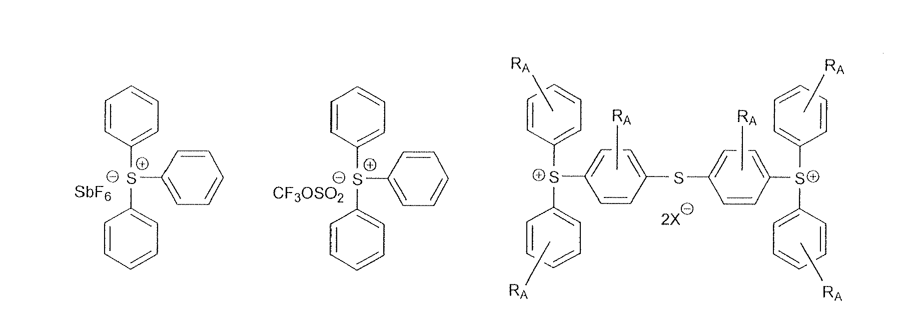 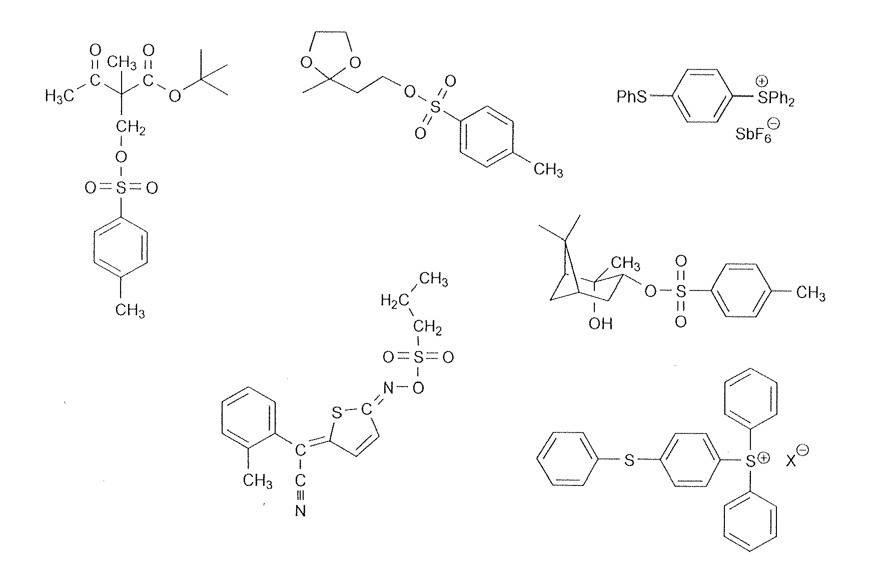 In the above formulas, Ar, Ar¹, and Ar² are substituted or unsubstituted aromatic groups having 6 to 20 carbon atoms, R, R¹, R², R³, and R_{A} are substituted or unsubstituted aromatic groups having 6 to 20 carbon atoms or substituted or unsubstituted aliphatic groups having 1 to 20 carbon atoms, and X, X_{A}, Y, and Z are aliphatic sulfonium groups, aliphatic sulfonium groups having fluorine, a tetrafluoroborate group, or hexafluorophosphonium group.

As the PBG, in addition to known compounds of which the structures are exemplified below, compounds having the same effect can be commonly used. These compounds can be appropriately selected according to the type of substrate, a desired shape, size, and the like of minute patterns. The amount of PBG added is usually in a range of 50 to 0.1 wt% of the total amount of the photoresist base material. However, when PBG is used as a chromophore, the amount of the PBG to be added is appropriately adjusted taking the amount of basic impurities contained in the base material into consideration, so that the reaction may not become uncontrollable due to an excessive amount of PBG. 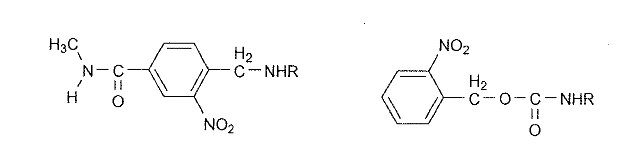 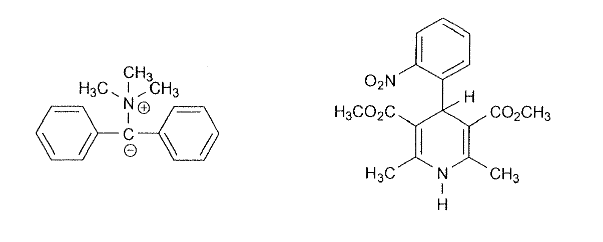 In the above formulas, R is a substituted or unsubstituted aromatic group having 6 to 20 carbon atoms or a substituted or unsubstituted aliphatic group having 1 to 20 carbon atoms.

When the composition of the invention contains a photoacid generator (PAG) or the like as a chromophore, the reaction may unnecessarily proceed due to generation of an excess amount of acids from the PAG, migration of the generated acids to areas other than desired areas in the photoresist thin films, and the like, resulting in impaired resolution. For this reason, when it is necessary to promote the performance as a photoresist, particularly resolution of the photoresist, a basic compound is optionally added as a quenching agent in addition to additives such as a PAG and the like. In addition, when a photobase generator (PBG) is included as a chromophore, an acid compound is added as a quenching agent.
Specifically, the quenching agent is defined as an additive to inhibit an overreaction of PAG or PBG.

As the quenching agent, in addition to known basic compounds and acidic compounds, other compounds having the same effect can be commonly used. These compounds can be appropriately adjusted according to the type of substrate, a desired shape, size, and the like of minute patterns. In the invention, it is preferable to use a basic organic compound or an acidic organic compound as a quenching agent from the viewpoint of solubility in the photoresist composition and dispersibility and stability in photoresist layers.

As specific examples of the basic organic compound, in addition to pyridines such as quinoline, indole, pyridine, and bipyridine, pyrimidines, pyrazines, piperidine, piperazine, pyrrolidine, 1,4-diazabicyclo[2.2.2]octane, aliphatic amines such as triethylamine, tetrabutylammonium hydroxide, and the like can be given. As specific examples of the acidic organic compound, p-toluenesulfonic acid, phenol, benzoic acid, and the like can be given.

The amount of the quenching agent to be added is usually from 25 to 1 × 10⁻⁷ wt% of the photoresist base material or from 50 to 0.01 wt% of the amount of PAG or PBG.

To the extent not impairing the effect of the invention, other additives may be optionally added to the photoresist composition of the invention in addition to PAG and PBG. Such additives include a base such as tetrabutylammonium hydroxide and its salt, an anti-light splitting agent, a plasticizer, a speed promoter, a photosensitizer, a sensitizer, an acid growth functional material, an etching resistance reinforcing agent, and the like.

One of these additives may be used alone, a mixture of two or more additives with the same or different functions may be used, or a mixture of precursors of these components may be used. The composition and the amounts incorporated of these components can be appropriately adjusted according to the desired shape, size, and the like of minute patterns. In general, the same composition ratio and the like as in conventional photoresist can be applied.

As the solvent, any solvents commonly used as a solvent for photoresist compositions can be used. Specific examples are glycols such as 2-methoxyethyl ether, ethylene glycol monomethyl ether(2-methoxyethanol), propylene glycol monomethyl ether, and acetoxymethoxypropane; lactates such as ethyl lactate and methyl lactate; propionates such as methyl propionate and ethyl propionate; cellosolve esterses such as methyl cellosolve acetate; aromatic hydrocarbonses such as toluene and xylene; ketones such as methyl ethyl ketone, cyclohexanone, and 2-heptanone; and the like. These solvents can be appropriately selected according solubility of the base material in the solvent, film-formability, and the like.

When the composition of the invention contains a solvent, the proportion of the components to the solvent is appropriately determined so that a photoresist layer with a desired thickness can be formed. Specifically, the proportion is generally from 1 to 40 wt% of the total amount of the composition. This proportion, however, can be appropriately adjusted according to the types of the base material and solvent, the thickness of the photoresist layer, and the like.

The composition of the invention is uniformly applied to a substrate such as a silicon wafer or any optional layers to be processed formed on a silicon wafer by spin coating, dip coating, painting, and the like. In a common practice after the application, the coated material is dried with heating at 80 to 160°C, for example, until the photoresist coating layer becomes non-sticky in order to remove the solvent. The heating conditions, however, can be appropriately adjusted according to the types of the base material and solvent, the thickness of the photoresist layer, and the like.

Next, the substrate on which the photoresist coating layer is no more sticky is exposed to extreme ultraviolet radiation or is irradiated with an electron beam by any optional method through a photomask to cause protective groups contained in the base material to dissociate, thereby producing solubility differences between the exposed areas and unexposed areas on the photoresist coating layer. After the exposure, the substrate is baked to increase the solubility differences, followed by development with an alkaline developer in order to form relief images. Patterns processed by ultra-microfabrication can be formed on the substrate in this manner.

If the photoresist base material and the composition thereof of the invention are used, patterns with isolated lines of 100 nm or less, particularly 50 nm or less, a 1:1 line-and-space, holes, etc, can be formed at a high sensitivity, high contrast, and low line edge roughness by ultra-microfabrication using extreme ultraviolet radiation or an electron beam.

### EXAMPLES

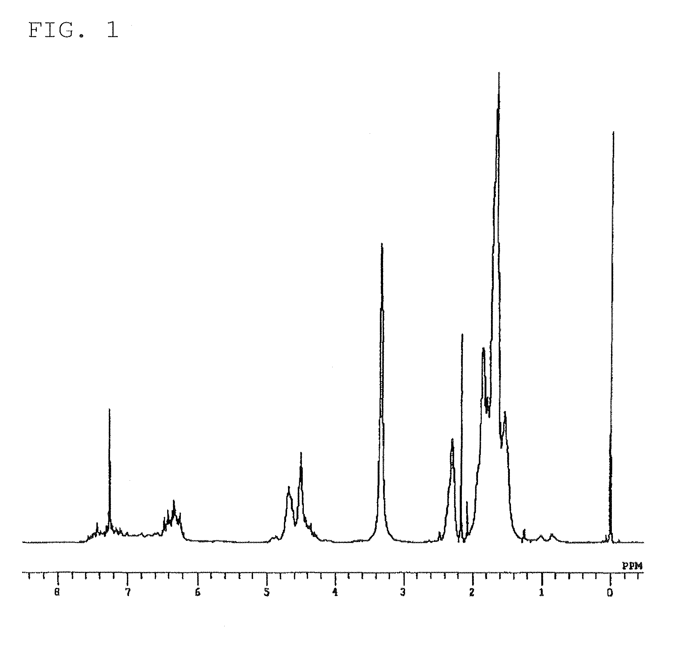

The invention is described more specifically by way of examples. However, the following examples should not be construed as limiting the invention.
Example 1
[Photoresist base material]
(1) Synthesis of calix-[4]-resorcinarene
   A three-neck flask (volume: 500 ml) equipped with a dripping funnel, a Dimroth condenser, and a thermometer, sufficiently dried and replaced with nitrogen gas, was encapsulated with resorcinol (33 g, 300 mmol) and acetaldehyde (17 ml, 300 mmol) in a nitrogen stream. Then, distilled methanol (300 ml) was added under a slight pressure of nitrogen gas to obtain a methanol solution. The methanol solution was heated at 75°C on a oil bath while stirring. 75 ml of a concentrated hydrochloric acid solution was slowly added by dripping from the dripping funnel, followed by continued stirring with heating at 75°C for two hours. After completion of the reaction, the mixture was allowed to cool to room temperature, followed by cooling on an ice water bath. The reaction mixture was allowed to stand for one hour. White raw crystals of the target compound were produced and collected by filtration. The crude crystals were washed twice with purified water (100 ml), purified by recrystallization from a mixed solution of ethanol and water, and dried under reduced pressure to obtain calix-[4]-resorcinarene (16 g, yield: 40.2%), which is a compound of the formula (1) with hydrogen atoms for all Rs. The structure of this compound was identified by NMR, IR, elementary analysis, and the like.
(2) Synthesis of calix-[4]-resorcinarene compound
   A two-neck flask (volume: 50 ml) equipped with a Dimroth condenser and a thermometer, sufficiently dried and replaced with nitrogen gas, was encapsulated with calix-[4]-resorcinarene (1.09 g, 2.0 mmol), a compound with hydrogen atoms for all Rs, prepared in (1) above, sodium carbonate (0.84g, 7. 9 mmol), and 15-crown-5 (0.63 g, 2. 9 mmol). The flask was replaced with nitrogen gas. Next, after adding 16 ml of acetone to prepare a solution, 2-methyl-2-adamantyl bromoacetate (1.52 g, 5.3 mmol) was added and the mixture was heated to reflux in a nitrogen atmosphere in an oil bath at 65°C while stirring for 24 hours. The reaction mixture was allowed to cool to room temperature and filtered. The filtrate was slowly added to 40 ml of a 0.5 M aqueous solution of acetic acid to obtain a white precipitate. The precipitate was collected by filtration, washed with purified water, and dried under reduced pressure to obtain a calixresorcinarene compound (2.27 g, yield: 100%), which is a compound of the formula (1) with a 2-methyl-2-adamantyloxycarbonylmethyl group for 36% of Rs and a hydrogen atom for 64% of Rs. This compound was used as a photoresist base material. The calixresorcinarene compound was analyzed by ¹H-NMR to determine the structure and the percentage of 2-methyl-2-adamantyloxycarbonylmethyl groups in Rs to confirm that the compound has the targeted structure. The ¹H-NMR spectrum chart is shown in Figure 1.

### Preparation Example 1

### [Photoresist base material]

### (1) Synthesis of calix-[4]-resorcinarene

A three-neck flask (volume: 500 ml) equipped with a dripping funnel, a Dimroth condenser, and a thermometer, sufficiently dried and replaced with nitrogen gas, was encapsulated with resorcinol (33 g, 300 mmol) and acetaldehyde (17 ml, 300 mmol) in a nitrogen stream. Then, distilled methanol (300 ml) was added under a slight pressure of nitrogen gas to obtain a methanol solution. The methanol solution was heated at 75°C on a oil bath while stirring. 75 ml of a concentrated hydrochloric acid solution was slowly added by dripping from the dripping funnel, followed by continued stirring with heating at 75°C for two hours. After completion of the reaction, the mixture was allowed to cool to room temperature, followed by cooling on an ice water bath. The reaction mixture was allowed to stand for one hour. White raw crystals of the target compound were produced and collected by filtration. The crude crystals were washed twice with purified water (100 ml), purified by recrystallization from a mixed solution of ethanol and water, and dried under reduced pressure to obtain calix-[4]-resorcinarene (16 g, yield: 40.2%), which is a compound of the formula (24) with hydrogen atoms for all Rs. The structure of this compound was identified by NMR, IR, elementary analysis, and the like.

### (2) Synthesis of calix-[4]-resorcinarene compound

A two-neck flask (volume: 100 ml) equipped with a Dimroth condenser and a thermometer, sufficiently dried and replaced with nitrogen gas, was encapsulated with calix-[4]-resorcinarene (2.07 g, 3.8 mmol) prepared in (1) above, potassium carbonate (7.32 g, 30 mmol), and 18-crown-6 (0.52 g, 1.94 mmol). The flask was replaced with nitrogen gas. Next, after adding 38 ml of acetone to prepare a solution, tert-butyl bromoacetate (3.51 g, 18 mmol) was added and the mixture was heated to reflux in a nitrogen atmosphere in an oil bath at 75°C while stirring for 24 hours. After completion of the reaction, the mixture was allowed to cool to room temperature and ice water was added, followed by stirring for one hour to obtain a white precipitate. The precipitate was collected by filtration and dried under reduced pressure to obtain a crude product of a calixresorcinarene compound (3.04 g, yield: 80%), which is a compound of the above formula (24) with a tert-butyloxycarbonylmethyl group for 50% of Rs and a hydrogen atom for 50% of Rs. In order to remove a light amount of potassium carbonate, the compound was dissolved in acetone (10 ml) and the resulting solution was added to an aqueous solution of acetic acid (1 mol/l, 300 ml) to obtain a white precipitate. The precipitate was collected by filtration and dried under reduced pressure to obtain a purified product of the above compound (2.58 g, purification yield: 85%). The structure of this compound was identified by TGA (weight of tert-butyloxycarbonylmethyl group dissociated near 170°C), NMR, IR, elementary analysis, and the like. In addition, as a result of qualitative analysis by inductively-coupled plasma source mass spectrometry, the amount of potassium ions contained in this compound was found to be 1,000 ppm to 1,500 ppm.

### (3) Purification of calix-[4]-resor_cinarene compound

The calixresorcinarene compound (2 g) obtained in (2) above was added to a 1 mol/l aqueous solution of acetic acid (100 ml) and the mixture was stirred at room temperature for three hours in a suspended state. The suspension was filtered and the recovered solid was washed three times with purified water (100 ml). The washed solid was dried at 80°C under reduced pressure to obtain a calixresorcinarene compound treated with an acetic acid aqueous solution. An acetone solution of the treated compound was passed through a glass column packed with a cation exchange resin (Amberlyst 15J-HG Dry, manufactured by Organo Corp.) which was previously replaced with acetone. The acetone solution recovered form the column was concentrated under vacuum and added to ultra purified water to reprecipitate the compound. The precipitate was dried at 80°C under reduced pressure to obtain an ion-exchange-treated compound (1.5 g, purification yield: 75%). This compound was used as a photoresist base material. As a result of qualitative analysis by inductively-coupled plasma source mass spectrometry, the amount of potassium ions contained in this base material was found to be 0.5 to 1.5 ppm.

### Example 2

### [Photoresist composition]

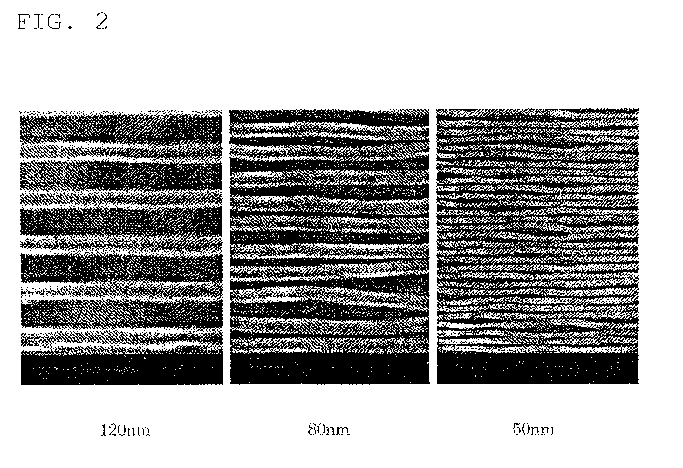

A solid mixture consisting of 87 parts by weight of calixresorcinarene compound obtained by washing with an acidic solution, treating with an ion-exchange resin, and removing basic impurities in Preparation Example 1(3), as a base material, 10 parts by weight of triphenylsulfonium trifluoromethanesulfonate, as a PAG, and 3 parts by weight of 1,4-diazabicyclo[2.2.2]octane, as a quenching agent, was dissolved in 2-methoxyethanol to a solid concentration of 20 wt%, thereby obtaining a photoresist solution (photoresist composition). The photoresist solution was applied onto a silicon wafer by spin coating (4,000 rpm, 60 sec) and baked at 90°C for 180 seconds to form a thin film with a thickness of 200 nm. The substrates to which the coating was applied were exposed to an electron beam at a dose of 20 µC/cm² using an electron beam exposure equipment ("JBX-5DR" manufactured by JEOL Ltd.) to draw line-and-space patterns with a line width and a line-to-line space of 50 nm, 80 nm, and 120 nm. After that, the substrate was baked at 90°C for 60 seconds, treated with a 2.38 wt% aqueous tetrabutylammonium hydroxide solution for 60 seconds, and washed with purified water. As a result, all line-and-space patterns were clearly drawn as shown in Figure 2.

### Comparative Example 1

### [Photoresist composition]

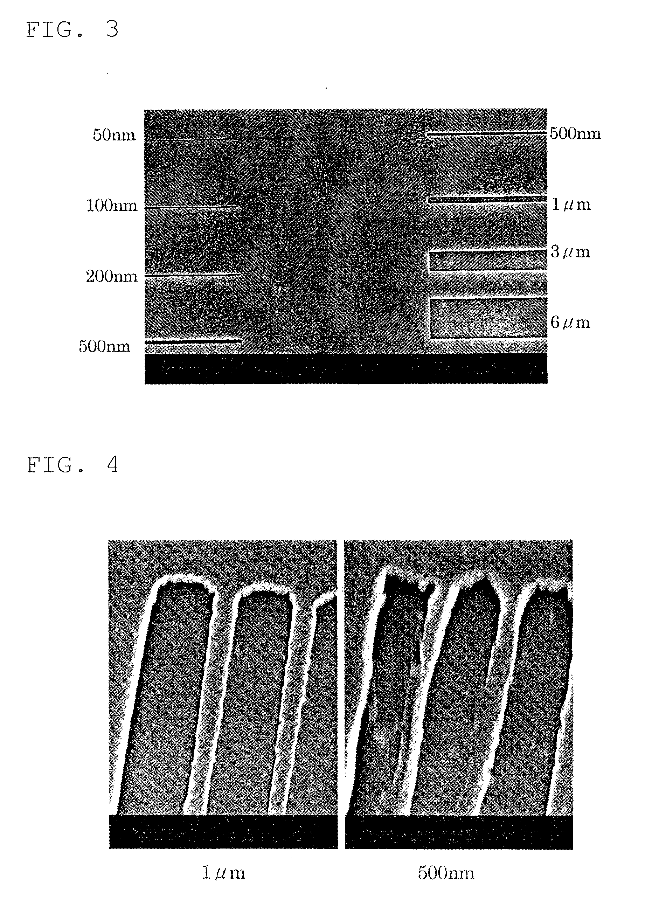

A photoresist solution was prepared by dissolving a calixresorcinarene compound from which basic impurities were removed in Preparation Example 1(3), as a base material, in 2-methoxy ethanol to the calixresorcinarene compound concentration of 20 wt%. The photoresist solution was applied onto a silicon wafer by spin coating (5000 rpm, 60 sec) and baked at 90°C for 180 seconds to form a thin film with a thickness of 500 nm. The substrates to which the coating was applied were exposed to an electron beam at a dose of 3,200 µC/cm² using an electron beam exposure equipment ("CABL9000" manufactured by CRESTEC Corp.) to draw isolated line patterns with a line with of 50 nm, 100 nm, 200 nm, 500 nm, 1 µm, 3 µm, and 6 µm. After that, the substrate was baked at 120°C for 60 seconds, treated with a 2.38 wt% aqueous tetrabutylammonium hydroxide solution for 60 seconds, and washed with purified water. As a result, all isolated patterns were clearly drawn as shown in Figure 3 in spite of a great dose of electron beam irradiation.

### Comparative Example 2

### [Photoresist composition]

A photoresist solution was prepared by dissolving a solid consisting of 90 parts by weight of the calixresorcinarene compound from which basic impurities were removed in Preparation Example 1(3), as a base material, and 10 parts by weight of triphenylsulfonium trifluoromethanesulfonate, as a PAG., in 2-methoxyethanol to a solid concentration of 20 wt%. The photoresist solution was applied onto a silicon wafer by spin coating (5000 rpm, 60 sec) and baked at 90°C for 180 seconds to form a thin film with a thickness of 500 nm. The substrates to which the coating was applied were exposed to an electron beam at a dose of 20 µC/cm² using an electron beam exposure equipment ("JBX-5DR" manufactured by JEOL Ltd.) to draw line-and-space patterns with a line width and a line-to-line space of 500 nm and 1 µm. After that, the substrate was baked at 110°C for 60 seconds, treated with a 2.38 wt% aqueous tetrabutylammonium hydroxide solution for 60 seconds, and washed with purified water. As a result, all line-and-space patterns were clearly drawn by a small dose of electron beam irradiation, although a structural disturbance is seen as shown in Figure 4.

### Comparative Example 3

### [Photoresist composition]

An experiment was carried out in the same manner as in Comparative Example 2, except for using a photoresist "ZEP520" manufactured by Zeon Corp. instead of the photoresist solution containing the calixresorcinarene compound. As a result, in all substrates, lines were not formed and the resist films were reduced in thickness. No patterns were formed at all.

### INDUSTRIAL APPLICABILITY

The photoresist base material and the composition thereof the invention is suitably used in the electricity and the electronic field such as a semiconductor device, the optical field, and the like. Performance of semiconductor devices such as ULSI can be outstandingly promoted by using the photoresist base material and the composition.

## Claims

1. A calixresorcinarene compound shown by the following formula (1), 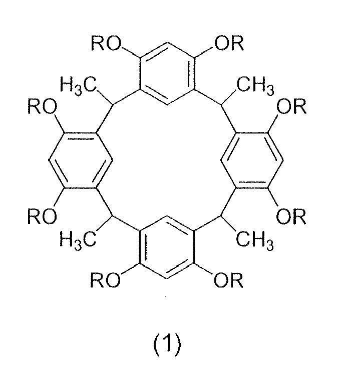 wherein R individually represents a hydrogen atom, a 1-tetrahydropyranyl group, a 1-tetrahydrofuranyl group, or one or more organic groups selected from the group consisting of the organic groups shown by the following formulas, 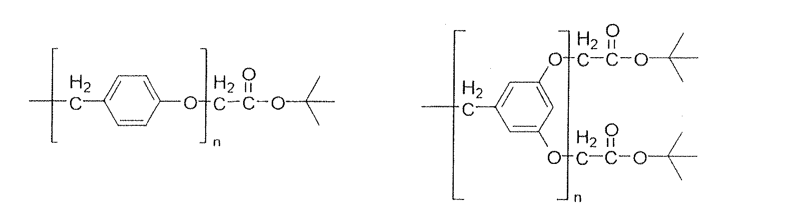 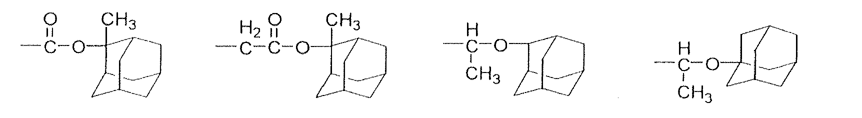 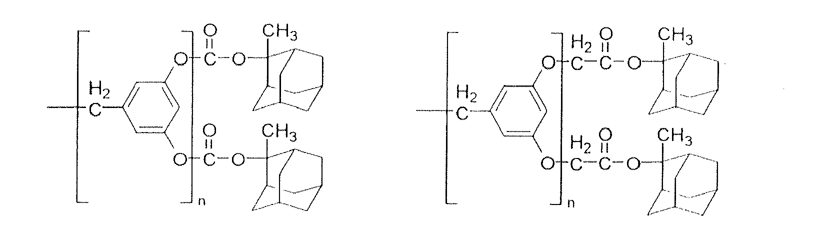 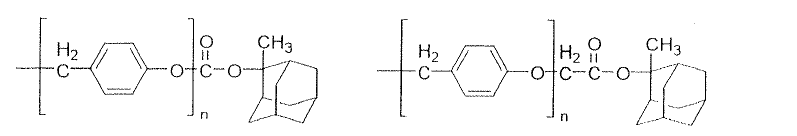 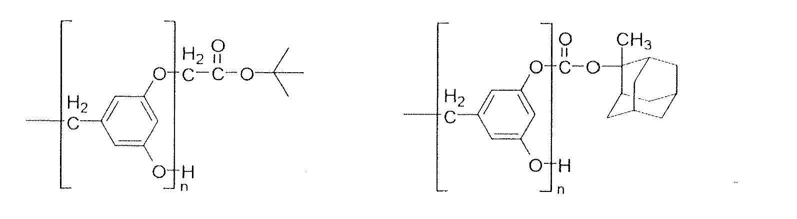 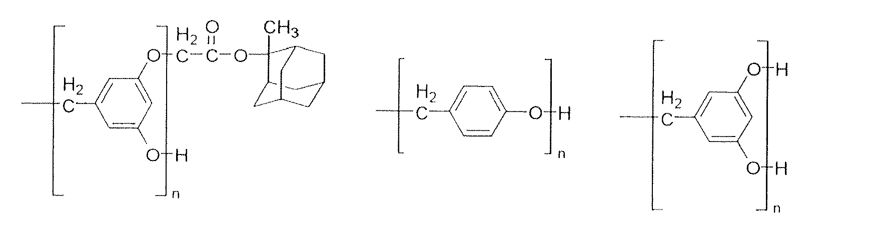 wherein n individually represents an integer of 1 to 50,
provided that a compound in which R is selected only from a hydrogen atom, a 1-tetrahydropyranyl group, and a 1-tetrahydrofuranyl group is excluded.

2. A purification method of a calixresorcinarene compound comprising washing the compound according to claim 1 with an acidic aqueous solution and processing the washed compound with an ion-exchange resin.

3. A photoresist base material for extreme ultraviolet radiation and/or an electron beam comprising the calixresorcinarene compound shown by the above formula (1).

4. A photoresist composition for extreme ultraviolet radiation and/or an electron beam comprising the photoresist base material according to claim 3 and a solvent.

5. The photoresist composition according to claim 4, further comprising a photoacid generator.

6. The photoresist composition according to claim 4 or 5, further comprising a basic organic compound as a quenching agent.

7. A photoresist composition comprising a photoresist base material that is an extreme ultraviolet radiation-reactive organic compound shown by the following formula (2), obtained by washing with an acidic aqueous solution and processing with an ion-exchange resin, a photoacid generator or a photobase generator, and a quenching agent, 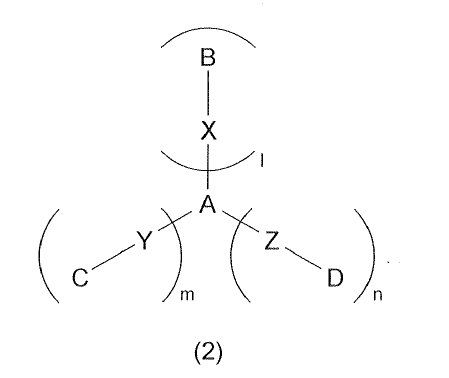 wherein A is an organic group represented by one of the following formulas, 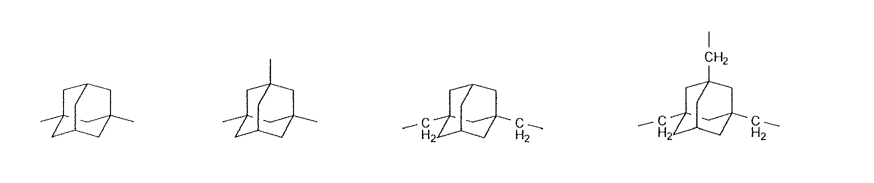 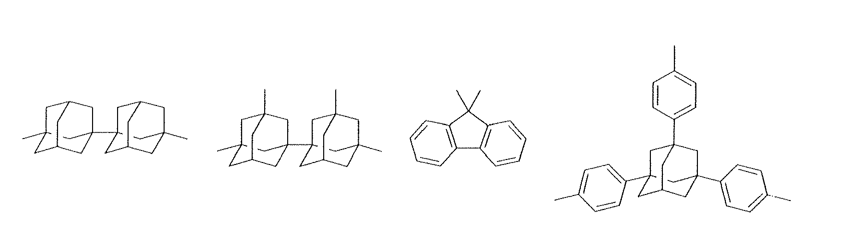 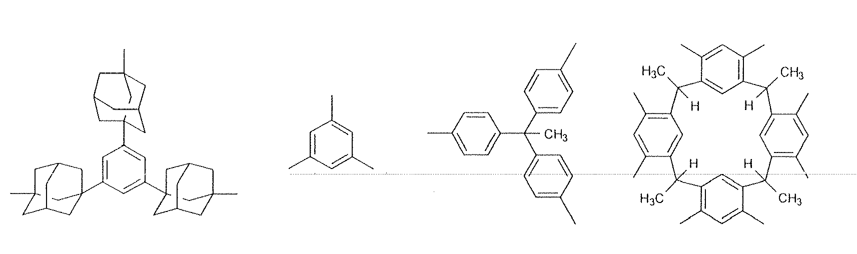 B, C, and D are individually a group reactive with extreme ultraviolet radiation, a group reactive with an effect of a chromophore active to extreme ultraviolet radiation, or an organic group of any of the following formulas, 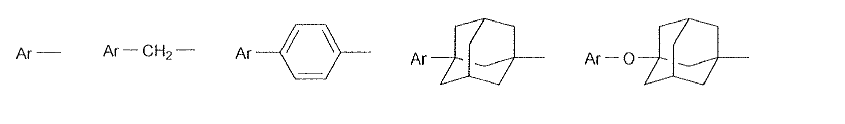 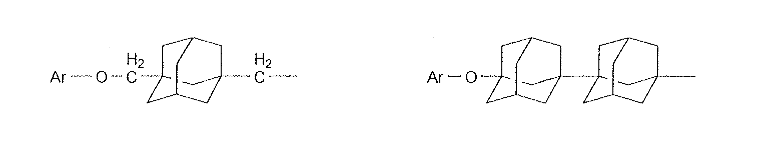 wherein Ar is a phenyl group or a naphthyl group substituted with RO- and/or ROCO-, wherein R, RO-, and ROCO- are groups reactive with extreme ultraviolet radiation or groups reactive with an effect of a chromophore active to extreme ultraviolet radiation,
X, Y, and Z individually represent a single bond or an ether bond, and 1 + m + n = 2, 3, 4, or 8.

8. The photoresist composition according to claim 7, wherein the extreme ultraviolet-radiation reactive organic compound is in an amorphous state at room temperature and the average diameter of the molecule is 2 nm or less.

9. The photoresist composition according to claim 7, wherein A is an organic group represented by any of the following formulas, 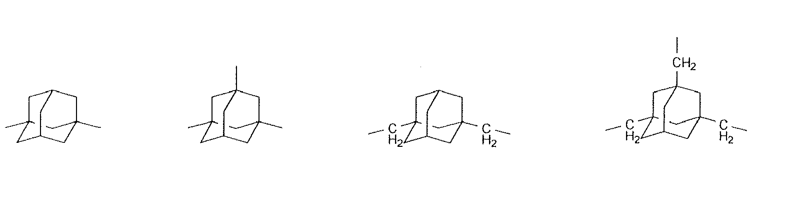 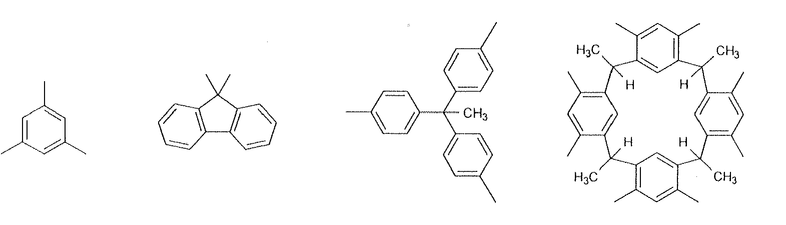 B, C, and D are individually a hydrogen atom, a tert-butyl group, tert-butyloxycarbonylmethyl group, tert-butyloxycarbonyl group, 1-tetrahydropyranyl group, 1-tetrahydrofuranyl group, 1-ethoxyethyl group, 1-phenoxyethyl group, an organic group shown by the following formula, 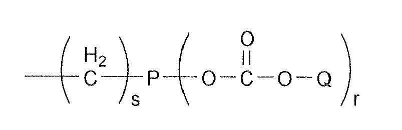 wherein P is an aromatic group having 6 to 20 carbon atoms with a valence of (r + 1), Q represents an organic group having 4 to 30 carbon atoms, r is an integer of 1 to 10, and s is an integer of 0 to 10, or an organic group represented by any of the following formulas, 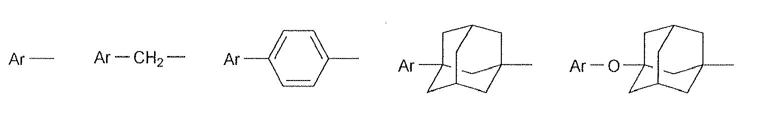 wherein Ar is a phenyl group or a naphthyl group substituted with RO- and/or ROCO-, wherein R is a hydrogen atom, a tert-butyl group, tert-butyloxycarbonylmethyl group, tert-butyloxycarbonyl group, 1-tetrahydropyranyl group, 1-tetrahydrofuranyl group, 1-ethoxyethyl group, 1-phenoxyethyl group, or an organic group shown by the following formula, 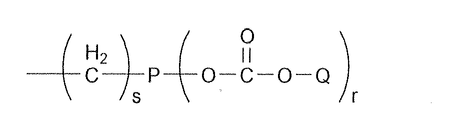 wherein P is an aromatic group having 6 to 20 carbon atoms with a valence of (r + 1), Q represents an organic group having 4 to 30 carbon atoms, r is an integer of 1 to 10, and s is an integer of 0 to 10, and
X, Y, and Z individually represent a single bond or an ether bond.

10. The photoresist composition according to claim 7, wherein A is any one of the organic groups represented by the following formulas, 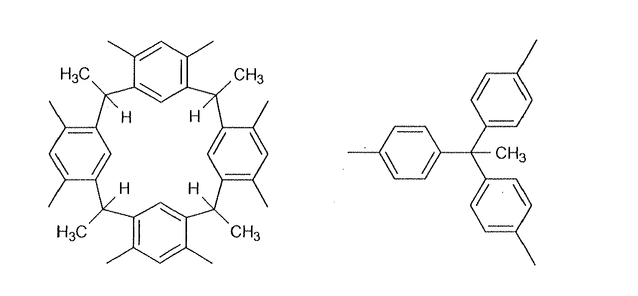 B, C, and D are individually a hydrogen atom, a tert-butyl group, tert-butyloxycarbonylmethyl group, tert-butyloxycarbonyl group, 1-tetrahydropyranyl group, 1-tetrahydrofuranyl group, 1-ethoxyethyl group, 1-phenoxyethyl group, or an organic group shown by the following formula, 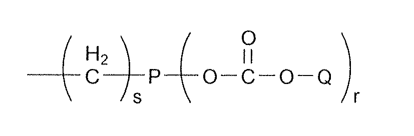 wherein P is an aromatic group having 6 to 20 carbon atoms with a valence of (r + 1), Q represents an organic group having 4 to 30 carbon atoms, r is an integer of 1 to 10, and s is an integer of 0 to 10, and
X, Y, and Z are ether bonds.

11. A photoresist composition comprising a photoresist base material that is a radiation-reactive organic compound shown by the following formula (2), obtained by washing with an acidic aqueous solution and processing with an ion-exchange resin, a photoacid generator or a photobase generator, and a quenching agent, 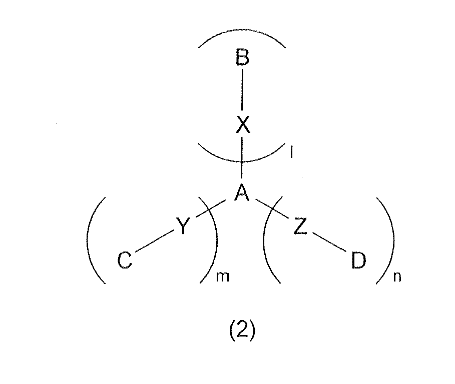 wherein A is an organic group represented by one of the following formulas, 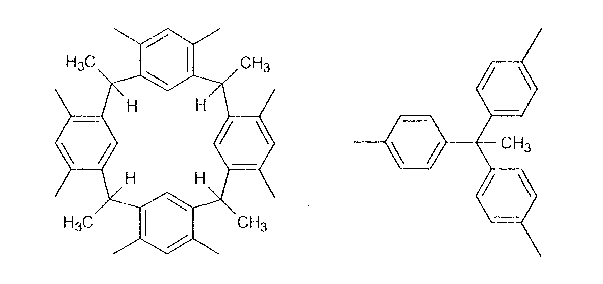 B, C, and D are individually a tert-butyloxycarbonylmethyl group, tert-butyloxycarbonyl group, or an organic group shown by the following formula, 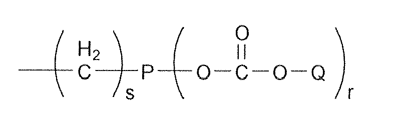 wherein P is an aromatic group having 6 to 20 carbon atoms with a valence of (r + 1), Q represents an organic group having 4 to 30 carbon atoms, r is an integer of 1 to 10, and s is an integer of 0 to 10, and
X, Y, and Z individually represent a single bond or an ether bond, and 1 + m + n = 3 or 8.

12. The photoresist composition according to claim 11, wherein the organic group shown by the following formula, 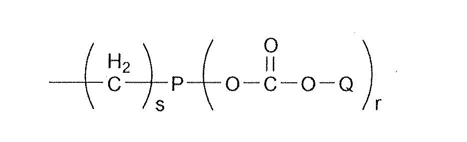 is a 4-(tert-butoxycarbonyloxy)benzyl group or a 3,5-di(tert-butoxycarbonyloxy)benzyl group.

13. The photoresist composition according to claim 11, wherein the radiation is extreme ultraviolet radiation or an electron beam.

14. The photoresist composition according to claim 7 or 11, wherein at least one of B, C, and D is a hydrogen atom and X, Y, and Z are ether bonds.

15. The photoresist composition according to claim 7 or 11, wherein the basic impurity content of the photoresist base material is not more than 10 ppm.

16. A microfabrication method by lithography using the photoresist composition according to claim 4, 7, or 11.

17. A semiconductor device prepared using the photoresist composition according to claim 4, 7, or 11.
